# EUROPEAN PATENT APPLICATION

(11) **EP 3 607 934 A1**
(43) Date of publication of application: **12.02.2020**
(21) Application number: 18781443.9
(22) Date of filing: 03.04.2018
(51) Int. Cl.: A61K 8/34, A61K 8/02, A61K 8/19, A61K 8/35, A61K 8/37, A61K 8/49, A61K 8/73, A61K 8/81, A61Q 1/00, A61Q 19/00, B05B 5/00

(54) **BODY COSMETIC**

(30) Priority: 04.04.2017 JP 2017074544
(71) Applicant: Kao Corporation, Chuo-Ku Tokyo 103-8210 (JP)
(72) Inventor: KIMURA, Shohei, Tokyo 131-8501 (JP)
(74) Representative: Hoffmann Eitle
(86) International application number: PCT/JP2018/014262
(87) International publication number: WO 2018/186394

(57) **Abstract**

A sheet-shaped body cosmetic comprising the following components (A), (B) and (C):
(A) from 500 to 20,000 ppm of carbon dioxide gas,
(B) from 0.15 to 3% by mass of a TRPM8 agonist, and
(C) water,
and the content of (D) ethanol being 5% by mass or less.

## Description

### Field of the Invention

The present invention relates to a body cosmetic.

### Background of the Invention

Long time standing job and desk work may cause fatigue, weariness, swelling or pain in each part of the body. It is widely known that promoting blood circulation by, for example, massage, stretching and taking a bath is effective for removing such symptoms.

For example, Patent Literature 1 discloses an agent for improving swelling, comprising a composition containing an agent for generating carbon dioxide gas capable of setting the concentration of carbon dioxide gas in hot water to 60 ppm or more. Patent Literature 1 describes that the agent is very effective for improving swelling in a short time by the action of carbon dioxide gas which vasodilates in a condition of high hydrostatic pressure.

It is also known that action of carbon dioxide gas on the skin promotes blood circulation in the skin even without water pressure on the body as in taking a bath. Then, studies are underway on, for example, cosmetics containing various types of carbon dioxide gas or substances which produce carbon dioxide gas using the above characteristics.

For example, Patent Literature 2 discloses a foaming liniment for promoting skin blood circulation containing hydrocarbon, carbon dioxide and emulsifier. Patent Literature 3 discloses a body cosmetic for promoting blood circulation containing carbon dioxide gas and TRPV3 agonist. Examples describe that attachment of a gel sheet containing carbon dioxide allows the effect of increasing blood flow to continue for 3 to 5 minutes after the attachment.

Furthermore, menthol is known to have the effect of promoting blood circulation (Non-patent Literature 1).

Meanwhile, ethanol, which can give refreshing feeling upon application and high antiseptic properties, has been used as a component suitable for mixing in various types of cosmetic. However, it is known that mixing ethanol in large amounts is undesirable from the viewpoint of skin irritation (Patent Literature 4).
(Patent Literature 1) JP-A-2005-314298
(Patent Literature 2) JP-A-2005-2046
(Patent Literature 3) JP-A-2012-167061
(Patent Literature 4) JP-A-2008-050272

(Non-patent Literature 1) Microvascular Research 107 (2016) 39-45

### Summary of the Invention

The present invention relates to a sheet-shaped body cosmetic comprising the following components (A), (B) and (C) :
(A) from 500 to 20,000 ppm of carbon dioxide gas,
(B) from 0.15 to 3% by mass of a TRPM8 agonist, and
(C) water,
and the content of (D) ethanol being 5% by mass or less.

### Brief Description of the Drawings

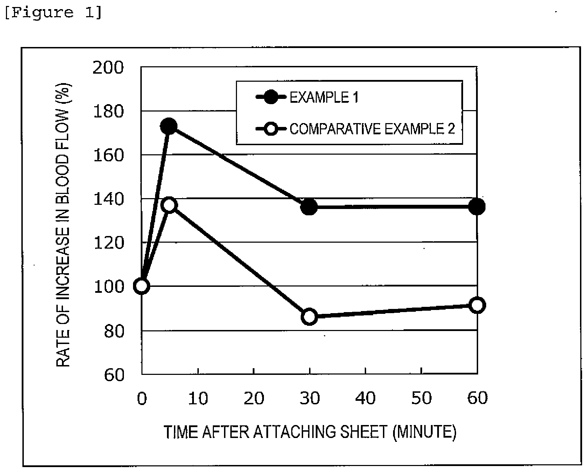

[Figure 1] Figure 1 is a graph showing the results for the rate of increase in blood flow in Example 1 and Comparative Example 2 in Examples.

### Detailed Description of the Invention

The agent for improving swelling of Patent Literature 1 was used in limited cases and was not convenient, because it was necessary to keep applying heat and high hydrostatic pressure for 5 minutes or more.

Furthermore, although Patent Literatures 2 and 3 utilize the effect of promoting blood circulation of carbon dioxide gas, since carbon dioxide gas diffuses into the air or metabolized in the living body immediately after application to the skin, the time of keeping increased blood flow after the application of the cosmetic was not enough to improve symptoms such as fatigue, weariness, swelling and pain of the body.

Furthermore, in actual cases it took time until the effect of promoting blood circulation caused by menthol as in Non-patent Literature 1 was exhibited and the effect was too small to improve symptoms such as fatigue, weariness, swelling and pain of the body.

The present invention provides a body cosmetic which can be applied to the body conveniently without limitation of time and place and which can increase and maintain the action of promoting blood circulation for a long time with low skin irritation.

The present inventor conducted intensive studies to solve the above problem, and as a result found that a body cosmetic which can be applied to the body conveniently and can increase and maintain the action of promoting blood circulation for a long time with low irritation can be obtained by combining a specific amount of carbon dioxide gas and a TRPM8 agonist and reducing the content of ethanol.

Due to the combination of carbon dioxide gas and a TRPM8 agonist, the body cosmetic of the present invention has an increased effect of promoting blood circulation, has an immediate and long lasting effect of promoting blood circulation, and offers comfortable tingling sensation, penetration sensation and effectiveness which have never been felt before. The body cosmetic also has excellent persistence of coolness and low skin irritation, and can be applied to the body conveniently without limitation of time and place.

The tingling sensation refers to a comfortable stimulation felt after the application of the body cosmetic, which means a feeling expecting that blood circulation is promoted.

Furthermore, the penetrating sensation means a feeling as if components of the body cosmetic penetrate deeply into the body after the application of the body cosmetic.

Hereinafter the present invention will be described in detail.

The form of the body cosmetic of the present invention is optional as described later. When the body cosmetic of the present invention is in the form of a gel sheet which includes a gel layer and a sheet substrate, the portion of the gel layer without the sheet substrate corresponds to the body cosmetic of the present invention.

Furthermore, the content of components other than component (A) means the content based on the total amount of the body cosmetic excluding component (A) unless otherwise specified.

The body cosmetic of the present invention increases and maintains the action of promoting blood circulation, and contains from 500 to 20,000 ppm of (A) carbon dioxide gas based on the total amount of the body cosmetic. The content of (A) carbon dioxide gas is preferably from 500 to 15,000 ppm, more preferably from 500 to 10,000 ppm, further preferably from 600 to 10,000 ppm, still more preferably from 700 to 3,000 ppm, yet more preferably from 800 to 3,000 ppm based on the total amount of the body cosmetic. A concentration of carbon dioxide gas of 500 ppm or more can give a sufficient action of promoting blood circulation, and can also improve the persistence of the action of promoting blood circulation when a TRPM8 agonist is used in combination. By contrast, a concentration of carbon dioxide gas of 20,000 ppm or less is preferred in manufacturing from the viewpoint of the solubility of carbon dioxide gas.

When the viscosity of the body cosmetic is low (1,000 Pa·s or less), the concentration of component (A) in the body cosmetic may be measured at 25°C by using a carbon dioxide gas electrode without diluting the body cosmetic. When the viscosity of the body cosmetic is high (1,000 Pa·s or more), the body cosmetic to be measured is put in a container with a lid containing an alkaline solution and sufficiently immersed therein to be trapped in the aqueous solution in the form of a carbonate, and then the solution is made acidic again using an acidic buffer; the concentration of carbon dioxide gas in the solution at a room temperature of 25°C is measured by using a carbon dioxide gas electrode, and then the concentration of carbon dioxide gas in the body cosmetic may be calculated based on the concentration of carbon dioxide gas obtained, the weight of the cosmetic and the volume of the solution.

The TRPM8 agonist, which is component (B) used in the present invention, acts on TRPM8, a cold receptor of the thermosensitive TRP channels existing in the body.

The TRPM8 agonist is not limited as long as it is a compound which activates TRPM8 channel. Examples thereof include menthol such as L-menthol and DL-menthol, menthanediol, cineol, menthyl glyceryl ether, menthyl lactate, menthyl succinate, menthyl glutarate, menthylpyrrolidone carboxylic acid, ethyl menthane carboxamide, menthanecarbonyl glycine ethyl ester, menthyl ethyl amino oxalic acid, menthone glycerin acetal, butylcyclohexanone, isopulegol, trimethyl isopropyl butanamide, menthoxypropanediol, camphor and icilin; plant and essential oil containing TRPM agonist, such as mint oil, peppermint oil, spearmint oil and eucalyptus oil may also be used. These may be used singly or in combinations of two or more. Of them, one or more selected from the group consisting of menthol, menthyl lactate, trimethyl isopropyl butanamide, menthoxypropanediol, camphor and icilin is preferred, and menthol is more preferred from the viewpoint of comfortable feeling and effectiveness upon application.

The content of component (B), the TRPM8 agonist, is 0.15% by mass or more, preferably 0.2% by mass or more, more preferably 0.3% by mass or more, further preferably 0.4% by mass or more based on the total amount of the body cosmetic from the viewpoint of improving the persistence of the effect of promoting blood circulation and obtaining comfortable tingling sensation when using carbon dioxide gas together. Furthermore, the content is preferably 3% by mass or less, more preferably 2% by mass or less, further preferably 1% by mass or less, still more preferably 0.8% by mass or less based on the total amount of the body cosmetic from the viewpoint of suppressing skin irritation.

In the present invention, preferably from 0.15 to 1% by mass, more preferably from 0.2 to 1% by mass, further preferably from 0.3 to 1% by mass, still more preferably from 0.4 to 0.8% by mass of menthol is included as component (B) based on the total amount of the body cosmetic.

The content of component (C), water, is preferably 60% by mass or more, more preferably from 65 to 95% by mass, further preferably from 70 to 90% by mass based on the total amount of the body cosmetic from the viewpoint of including carbon dioxide gas at high concentration.

The content of (D) ethanol in the body cosmetic of the present invention is 5% by mass or less based on the total amount of the body cosmetic. The content of ethanol is preferably as low as possible, more preferably less than 3% by mass based on the total amount of the body cosmetic, and it is further preferable that the body cosmetic does not contain ethanol with the concern that ethanol not only causes skin irritation but also facilitates rapid drying of the skin to cause chapped skin in people with hypersensitivity to ethanol. Although a very small or small amount (e.g., 0.001% by mass or more) of ethanol may be inevitably mixed, it is preferable that the content is as low as possible even in such cases. This allows the effect of promoting blood circulation of the body cosmetic to be maintained without increasing skin irritation.

The body cosmetic of the present invention may also contain (E) an oil agent, and this produces a higher effect of promoting blood circulation.

Such an oil agent is not particularly limited as long as it is used in usual cosmetic, and that which is liquid at 25°C is preferred. As used herein, being liquid means having fluidity at 25°C, and pastes are also included.

Examples of such an oil agent include a linear or branched hydrocarbon oil such as liquid paraffin, light isoparaffin, liquid isoparaffin, squalane and squalene; a silicone oil such as dimethylpolysiloxane, dimethylcyclopolysiloxane, methylphenylpolysiloxane, methylhydrogenpolysiloxane and higher alcohol modified organopolysiloxane; a mono ester oil such as isopropyl myristate, isononyl isononanoate and isotridecyl isononanoate; a diester oil such as neopentyl glycol dicaprate; a triester oil such as (caprylic/capric) triglyceride, 2-ethylhexanoic acid triglyceride and oleic acid triglyceride; and an ether oil such as alkyl-1,3-dimethylbutyl ether. These oil agents may be derived from a natural product.

Of them, (caprylic/capric) triglyceride, isopropyl myristate and isononyl isononanoate are preferred in consideration of compatibility with components (A) and (B) .

One or more of the oil agents of component (E) may be used. The content of the oil agent is preferably from 0.01 to 5% by mass, more preferably from 0.05 to 1% by mass based on the total amount of the body cosmetic from the viewpoint of increasing the effect of promoting blood circulation.

Furthermore, in the present invention, the mass ratio of component (B) to component (E), (B)/(E), is preferably from 0.1 to 50, more preferably from 0.5 to 20 form the viewpoint of penetration into the skin.

Other components usually used for cosmetics and drugs may be mixed in the body cosmetic of the present invention in addition to the above components according to the purpose of use, such as a powder component, a moisturizing agent, a water-soluble polymer, a thickener, a film forming agent, an ultraviolet absorber, a sequestrant, an alcohol, a saccharide, an amino acid derivative, an organic amine, a synthetic resin emulsion, a known blood circulation promoter, a skin nutrient, vitamin, an antioxidant, an auxiliary antioxidant, an antiseptic, a colorant and a fragrance.

Fragrances described in various references, e.g., "Synthetic Perfume, Chemistry and Knowledge on products" Motoichi Indo, 1996, The Chemical Daily Co., Ltd.; "Perfume and Flavor Chemicals," STEFFEN ARCTAMDER, 1969, are preferably used.

When applied to the skin, the body cosmetic of the present invention has a viscosity at 25°C of preferably 10 Pa·s or more, more preferably 1,000 Pa·s or more, further preferably from 2,000 to 100,000 Pa·s, still more preferably from 5,000 to 50,000 Pa·s from the viewpoint of convenience and comfortability upon application. The body cosmetic is packed in a container for measurement of viscosity and stored in a thermostat at 25°C for 24 hours, and then the viscosity is measured by using TV-10R type viscometer (VISCOMETER TVB-10 made by Toki Sangyo Co., Ltd.) with rotor T-F in the condition of a rotational speed of 1 rpm and a time of 1 minute.

The body cosmetic of the present invention has a pH of preferably 7.5 or less, more preferably from 4.5 to 7.0 from the viewpoint of exhibiting a good action of promoting blood circulation. The pH of the body cosmetic may be adjusted by using a pH adjuster so that the final pH falls within the above range. For example, an organic acid such as succinic acid, citric acid and tartaric acid, a salt thereof, phosphoric acid or a salt thereof is preferably used as the pH adjuster.

For the pH, when the viscosity is low (1,000 Pa·s or less), the value measured at 25°C of room temperature, without diluting the body cosmetic is determined as the pH of the body cosmetic of the present invention. When the viscosity is high (1,000 Pa·s or more), the value obtained by measurement at a room temperature of 25°C using a liquid component prepared by adding the body cosmetic to water in an amount 10 times the amount of the body cosmetic and sufficiently stirring the mixture, is determined as the pH of the body cosmetic of the present invention.

For the body cosmetic of the present invention, embodiments which may be adopted include an embodiment in which carbon dioxide gas is dissolved into the body cosmetic immediately before using the cosmetic, an embodiment, so-called disposable type, in which the body cosmetic is sealed in a sealed container such as an aluminum pillow package or an aerosol container so that carbon dioxide gas is previously dissolved into the body cosmetic, or an embodiment in which a carbonate and an acid is reacted to generate carbon dioxide gas so that carbon dioxide gas is dissolved into the body cosmetic.

The body cosmetic of the present invention is applied to the external skin all over the body. The body cosmetic is used in various applications including, in addition to so-called cosmetics, drugs and quasi-drugs. Examples include a facial or body cosmetic such as a sheet-shaped cosmetic, a cream, a lotion and a facial mask, a detergent, a gel, an ointment and a patch. Furthermore, the form of the body cosmetic of the present invention is not particularly limited, and may be a liquid, a lotion, an emulsion, a mist, a foam, a gel or a gel sheet depending on the purpose of use. The body cosmetic is preferably in the form of a foam, a gel or a gel sheet, more preferably a gel sheet from the viewpoint of keeping the body cosmetic on the surface of the skin for a long time so that the active ingredient penetrates the skin well to achieve a sufficient effect of promoting blood circulation.

For the portion to which the body cosmetic of the present invention is applied, the body cosmetic may be applied to the body excluding scalp, such as the leg, the arm, the shoulder and the waist. Since the body cosmetic of the present invention enables one to feel effectiveness such as tingling sensation and penetration sensation clearly, effectiveness can be felt strongly even in relatively insensitive portions, e.g., the leg including the calf, the ankle and the foot sole, and therefore the body cosmetic of the present invention is useful.

Furthermore, since the body cosmetic ensures an excellent effect of promoting blood circulation, the body cosmetic is useful as a body cosmetic for promoting blood circulation.

The body cosmetic of the present invention is used by directly applying it to the skin other than the scalp. More specifically, the body cosmetic of the present invention is applied to the surface of the skin of the body where symptoms such as fatigue, weariness, swelling and pain are felt.

It is preferable that at that time, a sufficient amount of the body cosmetic is applied thereto from the viewpoint of obtaining and maintaining the effect of increasing blood flow rate. More specifically, the thickness of the body cosmetic applied to the surface of the skin is preferably 0.5 mm or more, more preferably 0.7 mm or more, further preferably 1 mm or more. The thickness is preferably 3 mm or less, and more preferably 2 mm or less.

It is preferable that when the body cosmetic of the present invention is formed into a gel sheet, the body cosmetic of the present invention is used as a gel layer to form a sheet-shaped body cosmetic having the gel layer. The gel layer has a thickness of preferably 0.5 mm or more, more preferably 0.7 mm or more, further preferably 1 mm or more from the viewpoint of the effect of promoting blood circulation, retainability of carbon dioxide gas and easy attachment. The gel layer has a thickness of preferably 3 mm or less, more preferably 2 mm or less from the viewpoint of easy attachment.

The body cosmetic of the present invention is preferably in the form of a gel sheet from the viewpoint that it can be more easily used.

When the body cosmetic of the present invention is in the form of a gel sheet, the body cosmetic of the present invention may be produced by a method in which a sheet having a hydrogel layer containing components (B), (C) and (D) is placed in a pillow package and then the gas in the package is replaced with (A) carbon dioxide gas. The body cosmetic may also be produced by a method in which carbon dioxide gas is dissolved into gel at the time of or after forming a sheet, and the resulting sheet is placed in a pillow package, and then the gas in the package is removed or further replaced with carbon dioxide gas.

In this case the body cosmetic of the present invention is used as a hydrogel layer.

Furthermore, when the body cosmetic of the present invention is formed into a hydrogel layer, a body cosmetic sheet in which the hydrogel layer is supported by a sheet substrate may be formed. It is preferable that the water-containing gel layer is stacked on the sheet substrate. In this body cosmetic sheet, a peeling layer may also be stacked.

Any hydrogel layer may be used as long as the layer can retain water in a stable manner. It is preferable that the layer has a cross-linked structure formed from an anionic polymer, a cross-linking agent and water. It is preferable that a water-soluble anionic polymer is dissolved in water and cross-linked to form a hydrogel having a cross-linked structure of an anionic polymer, from the viewpoint of moldability.

The above cross-linked structure of an anionic polymer forms a dense network structure, which is the basic backbone of the hydrogel layer, by the crosslinking reaction of the anionic polymer, a cross-linking agent and water. The hydrogel layer formed by chemical cross-linking of an anionic polymer retains a large amount of water in the network structure, and is swelled, and thus has moderate elasticity, extensibility and flexibility. Furthermore, hydrogel formed by chemical cross-linking is less thermoplastic than, for example, agar gel, and thus exhibits stable shape-retainability in a wide range of operating temperature.

Examples of a water-soluble anionic polymer include polymers having a carboxyl group, a sulfate group or a phosphate group. Specific examples thereof include poly(meth)acrylic acid, carboxyvinyl polymer and a salt thereof; an anionic cellulose derivative and a salt thereof such as carboxymethylcellulose, and carboxyethylcellulose; carrageenan, alginic acid and a salt thereof, and an anionic starch derivative. It is preferable that at least one member selected from the group consisting of poly(meth)acrylic acid, carboxymethylcellulose, carrageenan and a salt thereof is included so as to obtain a hydrogel which achieves all of a large amount of water retained, sufficient gel strength and flexibility to follow irregularities and movement of the skin. Furthermore, carboxymethylcellulose, sodium carboxymethylcellulose, polyacrylic acid and sodium polyacrylate are more preferred from the viewpoint that they are industrially available at relatively low cost.

The content of the anionic polymer is preferably from 0.5 to 25% by mass, more preferably from 3 to 10% by mass based on the hydrogel layer, which is the body cosmetic of the present invention. Furthermore, the content of the anionic polymer is preferably from 0.5 to 6% by mass based on the hydrogel layer when a water-soluble polymer is mixed in order to adjust the viscosity of uncrosslinked gel and increase shape retainability of cross-linked gel by being physically entangled with the network structure of the anionic polymer.

A metal ion compound, a cationic polymer or a multifunctional epoxy compound, for example, may be used as the cross-linking agent. The cross-linking agent is selected based on the reactivity to the functional group of the anionic polymer to be used.

Examples of a metal ion compound include an oxide, a hydroxide and a salt containing aluminum, magnesium, calcium, potassium or the like, such as aluminum hydroxide, potassium alum, aluminum sulfate, aluminum oxide, aluminum glycinate, aluminum acetate, aluminum lactate, aluminum stearate, hydrated aluminum silicate, aluminum metasilicate, magnesium aluminometasilicate, magnesium chloride, magnesium stearate, calcium carbonate, calcium hydroxide, kaolin, synthetic hydrotalcite and potassium hydroxide. These may be mixed alone or in combination of two or more.

When carboxymethylcellulose or a salt thereof is selected as the anionic polymer, an aluminum ion compound such as magnesium aluminometasilicate and magnesium hydroxide is preferably used as a cross-linking agent.

The content of the cross-linking agent in the hydrogel layer is preferably from 0.01 to 10% by mass, more preferably from 0.05 to 5% by mass, further preferably from 0.1 to 2% by mass in the hydrogel layer.

Other components usually used for cosmetics and drugs may be mixed in the body cosmetic of the present invention (hydrogel layer) as necessary in addition to components (A) to (D), the anionic polymer and the cross-linking agent.

It is preferable that the hydrogel layer has a pH of 4.5 to 7.0 from the viewpoint of exhibiting a good action of promoting blood circulation.

The hydrogel layer has a thickness of preferably 0.5 mm or more, more preferably 0.7 mm or more, and further preferably 1 mm or more from the viewpoint of the effect of promoting blood circulation, retainability of carbon dioxide gas and easy attachment. The hydrogel layer has a thickness of preferably 3 mm or less, more preferably 2 mm or less from the viewpoint of easy attachment.

When the hydrogel layer, which is the body cosmetic of the present invention, is formed into a body cosmetic sheet in which the hydrogel layer is supported by a sheet substrate, woven fabric, nonwoven fabric, knitted fabric, synthetic resin film, waterproof paper and the like may be used as the sheet substrate, and a laminate thereof may also be used. More specifically, woven or nonwoven fabric of synthetic fiber such as nylon, polypropylene, polyester, polyethylene, polystyrene, polyurethane and polyolefin, or natural fiber such as silk, cotton, hemp, rayon and collagen; sheet of nylon, polypropylene, polyester, polyethylene, polyurethane and the like; and thin film sheet of pullulan or starch may be used. The sheet substrate has a thickness of about from 0.05 to 2.0 mm. The surface of the sheet substrate may be hydrophilized or hydrophobized.

In the body cosmetic sheet, a peeling layer may also be stacked. The peeling layer (sheet, film) may be stacked on the hydrogel layer on the side opposite to the support (sheet substrate) of the hydrogel layer in order to protect the surface to be attached. Examples of base materials of the peeling layer (sheet, film), which are not particularly limited, include polyester film such as polyethylene terephthalate film and polybutylene terephthalate film; polyolefin film such as polyethylene film and polypropylene film; and laminated paper prepared by laminating plastic such as polyethylene on craft paper, glassine paper, woodfree paper or the like. A peeling layer whose side has been hydrophobized with, for example, silicone, is more preferred. The peeling layer is stacked on the hydrogel with its hydrophobized surface facing the hydrogel.

More specifically, commercially available products such as Purex (Dupont Teijin Films), Cerapeel MF (Toray Advanced Film Co., Ltd.) and release paper (Lintec Corporation) may be used as the peeling layer.

It is preferable that the body cosmetic sheet is sealed in a pillow package together with carbon dioxide gas in order to dissolve carbon dioxide gas in a gel and keep carbon dioxide gas being dissolved. At that stage, the amount of carbon dioxide gas is 50% by mass or more, preferably 70% by mass or more, and further preferably 95% by mass or more based on the amount of gas in the pillow package from the viewpoint of maintaining the concentration of carbon dioxide gas dissolved in the gel.

When the content includes water, usually not only carbon dioxide gas but also nitrogen gas is used in a mixture; and the concentration of carbon dioxide gas is considered to be preferably from 20 to 30% by mass. The concentration is 70% by mass or less at most although it depends on the type of the content. In the present invention, the gas other than carbon dioxide gas is not particularly limited, and for example, air and nitrogen gas are preferred. The pressure of the gas in the pillow package is preferably from 0.8 to 2.0 atm, more preferably from 0.9 to 1.5 atm, further preferably from 0.9 to 1.2 atm at 25°C.

The pillow package needs to have poor permeability to carbon dioxide gas. As used herein, having poor permeability means a carbon dioxide gas permeability of 50 cc/ m²·day atm or less (ASTM D-1434). It is preferable that the pillow package is carbon dioxide gas impermeable. A material having heat sealing properties is preferred. Specific examples thereof include laminate film prepared by laminating aluminum foil, laminate film having aluminum deposited layer, polyvinylidene chloride film and laminate film having a polyvinylidene chloride layer. The form of the pillow package is preferably a flat bag or a gusset.

It is preferable that the pillow package has a volume such that the filling rate is maintained at preferably from 0.5 to 35%, more preferably from 1 to 25% at 25°C based on the volume calculated from its inner surface area. In this regard, conventional gas such as nitrogen gas, which is insoluble in an aqueous solution, has low thermal expansion; when carbon dioxide gas is dissolved in gel to saturation, carbon dioxide gas is not dissolved and is volatilized with an increase in temperature, causing the spatial volume to be almost doubled in some cases. For this reason, the pillow package preferably has a volume such that the filling rate is maintained at preferably from 1 to 10% at 25°C based on the volume calculated from its inner surface area.

The body cosmetic sheet produces the effect of promoting blood circulation when used by being attached to the body.

The calf, ankle, foot sole, shoulder, waist and arm, for example, are preferred as the portion of the body to which the body cosmetic sheet is applied. The time of use by attaching is not particularly limited, and is preferably 15 minutes or more, more preferably 30 minutes or more. For example, the sheet may be attached before sleep and continuously used during sleep.

For the embodiments described above, the present invention also discloses the following compositions and the like.
<1> A sheet-shaped body cosmetic comprising the following components (A), (B) and (C):
   (A) from 500 to 20,000 ppm of carbon dioxide gas,
   (B) from 0.15 to 3% by mass of a TRPM8 agonist, and
   (C) water,
   and the content of (D) ethanol being 5% by mass or less.
<2> A sheet-shaped body cosmetic comprising the following components (A), (B) and (C):
   (A) from 500 to 20,000 ppm of carbon dioxide gas,
   (B) from 0.2 to 3% by mass of a TRPM8 agonist, and
   (C) water,
   and the content of (D) ethanol being 5% by mass or less.
<3> The sheet-shaped body cosmetic according to <1> or <2> above, wherein component (B) is one or more selected from the group consisting of menthol, menthyl lactate, trimethyl isopropyl butanamide, menthoxypropanediol, camphor and icilin.
<4> The sheet-shaped body cosmetic according to any one of <1> to <3> above, wherein the content of component (C) is 60% by mass or more.
<5> The sheet-shaped body cosmetic according to any one of <1> to <4> above, wherein the sheet-shaped cosmetic comprises a gel layer.
<6> The sheet-shaped body cosmetic according to <5> above, wherein the gel layer has a thickness of 0.7 mm or more.
<7> The sheet-shaped body cosmetic according to any one of <1> to <6> above, wherein component (B) is menthol and the content thereof is from 0.15 to 1% by mass.
<8> The sheet-shaped body cosmetic according to any one of <1> to <6> above, wherein component (B) is menthol and the content thereof is from 0.2 to 1% by mass.
<9> The sheet-shaped body cosmetic according to any one of <1> to <6> above, wherein component (B) is menthol and the content thereof is from 0.3 to 1% by mass.
<10> The sheet-shaped body cosmetic according to any one of <1> to <6> above, wherein component (B) is menthol and the content thereof is from 0.4 to 0.8% by mass.
<11> The sheet-shaped body cosmetic according to any one of <1> to <10> above, further comprising (E) an oil agent.
<12> The sheet-shaped body cosmetic according to any one of <5> to <11> above, wherein the gel layer is a hydrogel layer comprising a cross-linked structure formed from an anionic polymer, a cross-linking agent and water.
<13> The sheet-shaped body cosmetic according to any one of <5> to <12> above, wherein the gel layer has a pH of from 4.5 to 7.0.
<14> The sheet-shaped body cosmetic according to any one of <5> to <13> above, wherein the gel layer is stacked on a sheet substrate.
<15> The sheet-shaped body cosmetic according to <14> above, wherein the sheet substrate is selected from the group consisting of woven fabric, nonwoven fabric, knitted fabric, synthetic resin film and waterproof paper.
<16> The sheet-shaped body cosmetic according to any one of <1> to <15> above, wherein the sheet-shaped body cosmetic comprises from 500 to 10,000 ppm of carbon dioxide gas of component (A).
<17> The sheet-shaped body cosmetic according to any one of <1> to <16> above, wherein the sheet-shaped body cosmetic comprises from 700 to 3,000 ppm of carbon dioxide gas of component (A).
<18> The sheet-shaped body cosmetic according to any one of <1> to <17> above, wherein the sheet-shaped body cosmetic comprises from 800 to 3,000 ppm of carbon dioxide gas of component (A).
<19> A sheet-shaped body cosmetic comprising the following components (A), (B) and (C):
   (A) from 500 to 20,000 ppm of carbon dioxide gas,
   (B) from 0.15 to 3% by mass of a TRPM8 agonist, and
   (C) water,
   and the content of (D) ethanol being 5% by mass or less, wherein the sheet-shaped body cosmetic comprises a hydrogel layer having a thickness of 0.7 mm or more and 3 mm or less.
<20> A sheet-shaped body cosmetic comprising the following components (A), (B) and (C):
   (A) from 500 to 20,000 ppm of carbon dioxide gas,
   (B) from 0.2 to 3% by mass of a TRPM8 agonist, and
   (C) water,
   and the content of (D) ethanol being 5% by mass or less, wherein the sheet-shaped body cosmetic comprises a hydrogel layer having a thickness of 0.7 mm or more and 3 mm or less.
<21> The sheet-shaped body cosmetic according to <19> or <20> above, wherein the sheet-shaped body cosmetic comprises from 800 to 3,000 ppm of carbon dioxide gas of component (A).
<22> The sheet-shaped body cosmetic according to any one of <19> to <21> above, wherein the hydrogel layer is stacked on a sheet substrate.
<23> The sheet-shaped body cosmetic according to any one of <19> to <22> above, further comprising (E) an oil agent.
<24> A sheet-shaped body cosmetic comprising the following components (A), (B) and (C):
   (A) from 500 to 3,000 ppm of carbon dioxide gas,
   (B) from 0.15 to 3% by mass of menthol, and
   (C) water,
   and the content of (D) ethanol being 5% by mass or less, wherein the sheet-shaped body cosmetic comprises a hydrogel layer having a thickness of 0.7 mm or more and 3 mm or less.
<25> A sheet-shaped body cosmetic comprising the following components (A), (B) and (C):
   (A) from 500 to 3,000 ppm of carbon dioxide gas,
   (B) from 0.2 to 3% by mass of menthol, and
   (C) water,
   and the content of (D) ethanol being 5% by mass or less, wherein the sheet-shaped body cosmetic comprises a hydrogel layer having a thickness of 0.7 mm or more and 3 mm or less.
<26> The sheet-shaped body cosmetic according to <24> or <25> above, wherein the hydrogel layer is stacked on a sheet substrate.
<27> The sheet-shaped body cosmetic according to any one of <24> to <26> above, wherein the hydrogel layer has a pH of 4.5 to 7.0.
<28> The sheet-shaped body cosmetic according to any one of <24> to <27> above, further comprising (E) an oil agent.
<29> Use of the sheet-shaped body cosmetic according to any one of <1> to <28> above for promoting blood circulation, comprising applying the sheet-shaped body cosmetic to any one of the calf, the ankle, the foot sole, the shoulder, the waist and the arm.
<30> A method for promoting blood circulation, comprising attaching the sheet-shaped body cosmetic according to any one of <1> to <28> above to any one of the calf, the ankle, the foot sole, the shoulder, the waist and the arm, and applying for 15 minutes or more.

### Examples

In the following, the present invention will be described in more detail with reference to Examples. The present invention is not limited to these Examples.

### Examples 1 to 6, Comparative Examples 1 to 5

A body cosmetic (gel sheet) was produced according to the formulation shown in Table 1 and evaluated for the rate of increase in blood flow, comfortability, tingling sensation, penetration sensation and skin irritation. The results are shown in Table 1. Furthermore, the results for the rate of increase in blood flow in Example 1 and Comparative Example 2 are shown in Figure 1.

The tingling sensation means a comfortable sensation of increased blood circulation at the portion of application. The penetration sensation means a sensation of deep penetration of components into the portion of application with a comfortable sensation of increased blood circulation in a deeper portion.

### (Method of production)

After preparing a raw liquid of uncrosslinked hydrogel according to the formulation shown in Table 1, a gel sheet was prepared. More specifically, carboxymethylcellulose, polyvinyl alcohol, dried aluminum hydroxide gel, glycerin and methyl paraoxybenzoate were mixed with an aqueous succinic acid solution, and the mixture was introduced into a kneader. Then, a TRPM8 agonist (component (B)) or, instead of component (B), propylene glycol in which a TRPV3 agonist (component(B') had been dissolved by heating, was added thereto and the mixture was mixed to prepare an uncrosslinked gel. The uncrosslinked gel obtained was sandwiched between polyethylene film and non-woven fabric, and the resultant was spread by a Baker type applicator so that the thickness of the water-containing gel was 1 mm. Furthermore, the same was aged at 50°C for 2 days to facilitate ion cross-linking reaction of the water-containing gel to give a cross-linked gel sheet. The gel sheet obtained was punched into a square (area 25 cm²) and inserted into an aluminum pillow package. The pillow package was sealed after replacing 90% or more of the air in the pillow package with carbon dioxide gas to set the concentration of carbon dioxide gas in the gel at about 1,000 ppm. The same was kept for 5 days and used as a gel sheet for evaluation. The aluminum pillow package was opened upon application and the gel sheet for evaluation was immediately attached to the skin. The action of increasing blood flow in the inner forearm and the sensation when the sheet was attached to the calf were evaluated based on the following criteria. In Example 6, the sheet was left in the air for about 1 minute after opening to adjust the concentration of carbon dioxide gas to about 750 ppm. In Comparative Example 3, a gel sheet without sealing of carbon dioxide gas was used. The pH of the gel sheets for evaluation was from 4.5 to 7.0.

### (Method of evaluation)

### (1) Rate of increase in blood flow:

After acclimation at a room temperature of about 25 ± 1°C and a humidity of about 50 ± 5% for 20 minutes, the blood flow in the inner forearm was measured by a laser speckle blood flow meter and determined as a value before application. The blood flow was measured 5 minutes, 30 minutes and 60 minutes after attaching each gel sheet to the inner forearm, and the rate of increase (%) was calculated based on the blood flow rate before application, which was taken as 100.

### (2) Comfortability

Comfortability was evaluated by three expert panelists 5 minutes, 30 minutes and 60 minutes after each gel sheet was attached to the calf based on the following criteria. The results are shown in an average value of the three panelists.
7: Very comfortable
6: Comfortable
5: Relatively comfortable
4: Moderate
3: Relatively unpleasant
2: Unpleasant
1: Very unpleasant

### (3) Tingling sensation, penetration sensation

Tingling sensation and penetration sensation were evaluated by three expert panelists 5 minutes, 30 minutes and 60 minutes after each gel sheet was attached to the calf based on the following criteria. The results are shown in an average value of the three panelists.
7: Very strongly felt
6: Strongly felt
5: Relatively strongly felt
4: Clearly felt
3: Felt
2: Slightly felt
1: Not felt at all

### (4) Skin irritation:

Skin irritation when each gel sheet was attached to the calf was evaluated by expert panelists based on the following criteria.
a: No irritation felt
b: Irritation slightly felt
c: Irritation felt

**[Table 1]**

| Component mixed (% by mass) | | | Comparative Example 1 | Example 1 | Example 2 | Example 3 | Example 4 | Example 5 | Example 6 | Comparative Example 2 | Comparative Example 3 | Comparative Example 4 | Comparative Example 5 |
|---|---|---|---|---|---|---|---|---|---|---|---|---|---|
| | Carboxymethylcellulose | | 3.5 | 3.5 | 3.5 | 3.5 | 3.5 | 3.5 | 3.5 | 3.5 | 3.5 | 3.5 | 3.5 |
| | Polyvinyl alcohol | | 3 | 3 | 3 | 3 | 3 | 3 | 3 | 3 | 3 | 3 | 3 |
| | Dried aluminum hydroxide | | 0.15 | 0.15 | 0.15 | 0.15 | 0.15 | 0.15 | 0.15 | 0.15 | 0.15 | 0.15 | 0.15 |
| | Succinic acid | | 0.4 | 0.4 | 0.4 | 0.4 | 0.4 | 0.4 | 0.4 | 0.4 | 0.4 | 0.4 | 0.4 |
| | Methyl paraoxybenzoate | | 0.25 | 0.25 | 0.25 | 0,25 | 0.25 | 0.25 | 0.25 | 0.25 | 0.25 | 0.25 | 0.25 |
| | Glycerin | | 5 | 5 | 5 | 5 | 5 | 5 | 5 | 5 | 5 | 5 | 5 |
| | Propylene glycol | | 5 | 5 | 5 | 5 | 5 | 5 | 5 | 5 | 5 | 5 | 5 |
| (B) | Menthol | | | 0.4 | 0.8 | 2 | | | 0.4 | 0.1 | 0.4 | 0.4 | |
| | Menthyl lactate | | | | | | 2 | | | | | | |
| | N,2,3-trimethyl-2-isopropyl butanamide | | | | | | | 2 | | | | | |
| (B') | Limonene | | | | | | | | | | | | 2 |
| (C) | Purified water | | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 |
| (A) | Concentration of carbon dioxide gas (ppm) | | 1000 | 1000 | 1000 | 1000 | 1000 | 1000 | 750 | 1000 | 0 | 100 | 1000 |
| | Rate of increase in blood flow (%) | After 5 minutes | 131 | 173 | 170 | 151 | 143 | 162 | 149 | 137 | 97 | 106 | 189 |
| | | After 30 minutes | 86 | 136 | 154 | 300 | 150 | 112 | 110 | 86 | 134 | 130 | 103 |
| | | After 60 minutes | 85 | 136 | 137 | 327 | 151 | 111 | 139 | 91 | 129 | 109 | 145 |
| | Comfortability | After 5 minutes | 4.3 | 5.3 | 5.7 | 5.7 | 4.7 | 4.7 | 5.0 | 4.0 | 4.3 | 4.3 | 5.0 |
| | | After 30 minutes | 4.0 | 6.3 | 6.3 | 6.7 | 6.7 | 5.3 | 6.3 | 5.0 | 5.7 | 5.7 | 4.0 |
| | | After 60 minutes | 4.0 | 5.3 | 5.3 | 5.3 | 5.7 | 6.0 | 5.3 | 4.0 | 4.7 | 5.0 | 4.0 |
| | Tingling sensation | After 5 minutes | 1.0 | 1.3 | 1.7 | 2.3 | 1.3 | 1.3 | 1.3 | 1.0 | 1.0 | 1.0 | 1.0 |
| | | After 30 minutes | 1.0 | 4.3 | 5.0 | 6.0 | 4.3 | 4.0 | 5.0 | 1.3 | 2.7 | 2.7 | 1.3 |
| | | After 60 minutes | 1.0 | 3.3 | 3.3 | 4.3 | 2.7 | 3.3 | 3.3 | 1.0 | 2.0 | 2.0 | 1.0 |
| | Penetration sensation | After 5 minutes | 1.0 | 1.7 | 1.7 | 1.7 | 1.3 | 1.3 | 1.3 | 1.0 | 1.3 | 1.3 | 1.0 |
| | | After 30 minutes | 1.0 | 4.3 | 4.7 | 5.0 | 5.0 | 4.3 | 4.7 | 1.7 | 2.3 | 2.3 | 1.3 |
| | | After 60 minutes | 1.0 | 2.0 | 2.0 | 3.0 | 4.3 | 4.3 | 2.0 | 1.0 | 1.7 | 1.7 | 1.0 |
| | Skin irritation | | a | a | a | b | a | a | a | a | a | a | c |

## Claims

1. A sheet-shaped body cosmetic comprising the following components (A), (B) and (C):
(A) from 500 to 20,000 ppm of carbon dioxide gas,
(B) from 0.15 to 3% by mass of a TRPM8 agonist, and
(C) water,
and the content of (D) ethanol being 5% by mass or less.

2. The sheet-shaped body cosmetic according to claim 1, wherein the content of the component (B) is from 0.2 to 3% by mass.

3. The sheet-shaped body cosmetic according to claim 1 or 2, wherein the component (B) is one or more selected from the group consisting of menthol, menthyl lactate, trimethyl isopropyl butanamide, menthoxypropanediol, camphor and icilin.

4. The sheet-shaped body cosmetic according to any one of claims 1 to 3, wherein the content of the component (C) is 60% by mass or more.

5. The sheet-shaped body cosmetic according to any one of claims 1 to 4, wherein the sheet-shaped cosmetic comprises a gel layer.

6. The sheet-shaped body cosmetic according to claim 5, wherein the gel layer has a thickness of 0.7 mm or more.

7. The sheet-shaped body cosmetic according to any one of claims 1 to 6, wherein the component (B) is menthol and the content thereof is from 0.2 to 1% by mass.

8. The sheet-shaped body cosmetic according to any one of claims 1 to 7, further comprising (E) an oil agent.

9. The sheet-shaped body cosmetic according to any one of claims 5 to 8, wherein the gel layer is a hydrogel layer comprising a cross-linked structure formed from an anionic polymer, a cross-linking agent and water.

10. The sheet-shaped body cosmetic according to any one of claims 5 to 9, wherein the gel layer has a pH of 4.5 to 7.0.

11. The sheet-shaped body cosmetic according to any one of claims 5 to 10, wherein the gel layer is stacked on a sheet substrate.

12. Use of the sheet-shaped body cosmetic according to any one of claims 1 to 11 for promoting blood circulation, comprising applying the sheet-shaped body cosmetic to any one of the calf, the ankle, the foot sole, the shoulder, the waist and the arm.

13. A method for promoting blood circulation, comprising attaching the sheet-shaped body cosmetic according to any one of claims 1 to 11 to any one of the calf, the ankle, the foot sole, the shoulder, the waist and the arm, and applying for 15 minutes or more.
